# EUROPEAN PATENT APPLICATION

(11) **EP 4 775 184 A1**
(43) Date of publication of application: **15.07.2026**
(21) Application number: 24862879.4
(22) Date of filing: 05.09.2024
(51) Int. Cl.: A61F 9/007

(54) **TROCAR FOR OPHTHALMIC SURGERY AND TROCAR SYSTEM INCLUDING TROCAR FOR OPHTHALMIC SURGERY**

(30) Priority: 06.09.2023 JP 2023144317
(71) Applicant: Hisatomi, Toshio, Gifu-shi, Gifu 501-1194 (JP)
(72) Inventor: Hisatomi, Toshio, Gifu-shi, Gifu 501-1194 (JP)
(74) Representative: Serjeants LLP
(86) International application number: PCT/JP2024/031920
(87) International publication number: WO 2025/053230

(57) **Abstract**

There is provided a trocar for ophthalmic surgery having a hollow insertion section 4 at a front side connected to a hollow main body section 6 at a rear side, forming an internal space 8 from a front opening 4A of the insertion section 4 to a rear opening 6A of the main body section 6, wherein at least the insertion section is elastic, wherein when a knife 20 is placed in the internal space 8 so that a cutting tip portion 22 of the knife 20 protrudes from the front opening 6A, and the knife 20 is viewed from the distal end of the cutting tip portion 22, an outline of the insertion section 4 is generally hidden by an outline of the cutting tip portion 22, when the cutting tip portion 22 is inserted into an interior of the eyeball, the insertion section 4 is also inserted into the interior of the eyeball together with the cutting tip portion 22, and by removing only the knife 20 from the rear opening 6A, an installed state is established in which the insertion section 4 is located inside the eyeball and the main body section 6 is located outside the eyeball, wherein the insertion section 4 expands, thereby allowing the cutting tip portion 22 to pass through the insertion section 4, wherein in the installed state, when a surgical instrument 30 is inserted into the insertion section 4 through the rear opening 6A, the insertion section 4 expands and a tip of the surgical instrument 30 is inserted into the eyeball; and when the surgical instrument 30 is removed from the rear opening 6A, the expanded insertion section 4 returns to its original shape. By providing the trocar for ophthalmic surgery 2 and a trocar system including this trocar for ophthalmic surgery 6, it is possible to reliably insert a surgical instrument having an outer shape larger than forceps into the eyeball, regardless of the skill of the surgeon.

## Description

### [Technical Field]

The present invention relates to a trocar for ophthalmic surgery used for inserting a surgical instrument into the eyeball during ophthalmic surgery.

### [Background Art]

In ophthalmic surgery, for example, as shown in Patent Document 1, a trocar with a thin metal tube is inserted into the surface of the eyeball during vitreoretinal surgery. Forceps are passed through the trocar, and the tip of the forceps can be inserted into the eyeball. Furthermore, the tip of the forceps can be brought to the fundus to perform retinal surgery. In the surgery described in Patent Document 1, the forceps have a diameter of approximately 0.5 mm, and since the forceps pivot around the insertion point of the trocar as a fulcrum, the outer diameter of the trocar is also very small. This makes it possible to insert the metal trocar into the eyeball.

### [Prior Art Documents]

### [Patent Documents]

[Patent Document 1] JP 2022-142902 A

### [Summary of the Invention]

### [Problem to be Solved by the Invention]

However, such trocars cannot be used with larger surgical instruments, such as ultrasonic handpieces used in phacoemulsification and aspiration of cataracts. If the outer diameter of a metal trocar were expanded to accommodate an ultrasonic handpiece, it would be difficult to insert it into the eyeball without damaging it, thereby deforming the wound and making it difficult to close.

Therefore, currently, surgeons use a knife to incise the tissue and create a wound, then insert the ultrasonic handpiece into the eyeball. In this case, the large outer diameter of the ultrasonic handpiece results in making a large wound. Furthermore, the surgical procedure depends on the surgeon's skill, and irregular wound openings and size discrepancies can make the surgical procedure unstable and lead to various complications. Furthermore, each time the handpiece is inserted or removed, aqueous humor leaks from the eyeball, thereby causing fluctuations in intraocular pressure and temporarily collapsing or expanding the eyeball, which can apply excessive force to areas inside the eyeball and potentially lead to complications.

Then, an object of the present invention is therefore to provide a trocar for ophthalmic surgery that enables surgical instruments with larger external dimensions than forceps to be reliably inserted into the eyeball, regardless of the surgeon's skill, and a trocar system including this Trocar for Ophthalmic Surgery.

### [Means for Solving the Problem]

One aspect of the present invention is a trocar for ophthalmic surgery having a hollow insertion section at a front side connected to a hollow main body section at a rear side, forming an internal space from a front opening of the insertion section to a rear opening of the main body section,
wherein at least the insertion section is elastic,
wherein when a knife is placed in the internal space so that a cutting tip portion of the knife protrudes from the front opening, and the knife is viewed from the distal end of the cutting tip portion, an outline of the insertion section is generally hidden by an outline of the cutting tip portion, when the cutting tip portion is inserted into an interior of the eyeball, the insertion section is also inserted into the interior of the eyeball together with the cutting tip portion, and by removing only the knife from the rear opening, an installed state is established in which the insertion section is located inside the eyeball and the main body section is located outside the eyeball,
wherein the insertion section expands, thereby allowing the cutting tip portion to pass through the insertion section,
wherein in the installed state, when a surgical instrument is inserted into the insertion section through the rear opening, the insertion section expands and a tip of the surgical instrument is inserted into the eyeball; and when the surgical instrument is removed from the rear opening, the expanded insertion section returns to its original shape.

Another aspect of the present invention is a trocar system including trocar for ophthalmic surgery comprising:
the above mentioned trocar for ophthalmic surgery,
the surgical instrument being an ultrasonic handpiece including an ultrasonic transducer having a suction port for aspirating irrigation fluid and fragmentation sections, and
an infusion trocar for supplying irrigation fluid,
wherein the trocar for ophthalmic surgery is heat resistant.

Still another aspect of the present invention is a trocar system including trocar for ophthalmic surgery comprising:
the above mentioned trocar for ophthalmic surgery,
the surgical instrument being an ultrasonic handpiece including an ultrasonic transducer having a suction port for aspirating irrigation fluid and fragmentation sections, and
an infusion trocar for supplying irrigation fluid,
wherein the trocar for ophthalmic surgery is heat resistant.

As described above, the present invention provides a trocar for ophthalmic surgery that enables reliable insertion of surgical instruments larger in outer dimensions than forceps into the eyeball, regardless of the surgeon's skill, and a trocar system including this trocar for ophthalmic surgery.

### [Brief Description of the Drawings]

[Figure 1] A perspective view showing an outer shape of a trocar for ophthalmic surgery according to a first embodiment of the present invention, where (a) is a view from the tip end and (b) is a view from the rear end
[Figure 2A] A view taken along the arrows A-A in Figure 1(a), showing a side view of the trocar for ophthalmic surgery from the rear end
[Figure 2B] A view showing a state that a knife or surgical instrument has been inserted into the trocar for ophthalmic surgery from the state shown in Figure 2A
[Figure 3] A cross-sectional view taken along the line B-B in Figure 2A, showing a side cross-section of the trocar for ophthalmic surgery and a state that a knife has been attached to the trocar for ophthalmic surgery
[Figure 4A] A view showing the maximum longitudinal dimension Lt1 of the trocar for ophthalmic surgery and the maximum width dimension Ln1 of the attached knife
[Figure 4B] A view showing the maximum lateral dimension Lt2 of the trocar for ophthalmic surgery and the maximum thickness dimension Ln2 of the attached knife
[Figure 5A] A schematic view showing a state that the insertion section of the trocar for ophthalmic surgery has been inserted into the interior of the eyeball by inserting the cutting tip portion of the attached knife into the interior of the eyeball
[Figure 5B] A schematic view showing a state that only the knife has been removed from the state shown in Figure 5A
[Figure 5C] A schematic view showing insertion of a surgical instrument into the trocar for ophthalmic surgery with the insertion section inserted inside the eyeball to perform surgery
[Figure 6] A schematic view showing performance of eye surgery using a trocar system including trocar for ophthalmic surgery including the trocar for ophthalmic surgery according to the first embodiment of the present invention
[Figure 7] A side cross-sectional view of a trocar for ophthalmic surgery according to a second embodiment of the present invention, particularly showing an insertion, section inserted inside the eyeball and insertion of surgical instruments to perform surgery
[Figure 8A] A view showing an example of an infusion trocar to be used with the trocar for ophthalmic surgery according to the second embodiment of the present invention, showing the irrigation fluid tube attached to the internal space of the main body in a collapsed state.
[Figure 8B] A view showing an example of an infusion trocar used in conjunction with the trocar for ophthalmic surgery according to the second embodiment of the present invention, showing supply of irrigation fluid into a folded irrigation fluid tube to supply fluid into the eyeball
[Figure 9] A view schematically showing performance of eye surgery using a trocar system including trocar for ophthalmic surgery including a trocar for ophthalmic surgery and the infusion trocar according to the second embodiment of the present invention
[Figure 10A] A perspective view schematically showing an example of a side port seal attached to a surgical auxiliary instrument
[Figure 10B] A perspective view schematically showing another example of a side port seal attached to a surgical auxiliary instrument
[Figure 11A] A perspective view schematically showing an example of a contact lens that covers the eyeball during surgery, used in conjunction with the trocar for ophthalmic surgery according to the first embodiment of the present invention
[Figure 11B] A perspective view schematically showing an example of a contact lens that covers the eyeball during surgery and is used together with the trocar for ophthalmic surgery according to the second embodiment of the present invention

### [Embodiment for carrying out the Invention]

Embodiments of the present invention will be described below with reference to the drawings. The embodiments described below embody the technical concepts of the present invention and, unless otherwise specified, do not limit the present invention to the following. In the drawings, components having the same function may be designated by the same reference numerals. For convenience, separate embodiments may be shown to facilitate explanation or understanding of key points. However, partial substitution or combination of configurations shown in different embodiments is possible. In the following embodiments, descriptions of features common to the previous embodiments will be omitted, and only differences will be described. In particular, similar effects resulting from similar configurations will not be mentioned in each embodiment. The size and positional relationships of components shown in the drawings may be exaggerated for clarity.

### (Trocar for Ophthalmic Surgery according to first embodiment of the present invention)

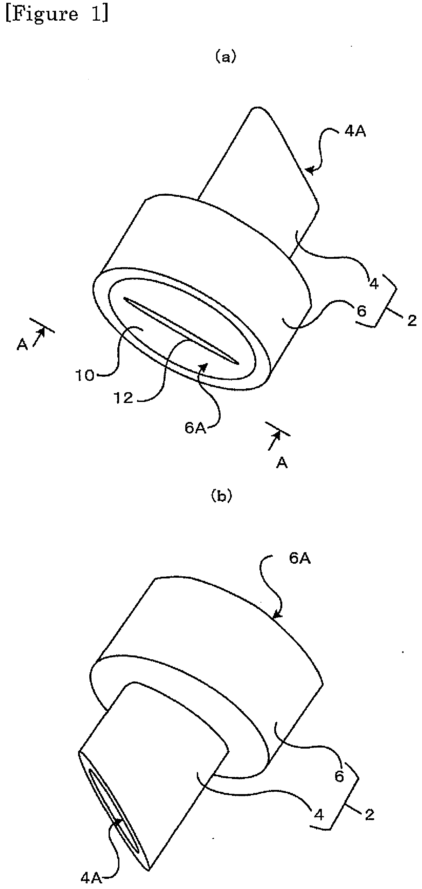

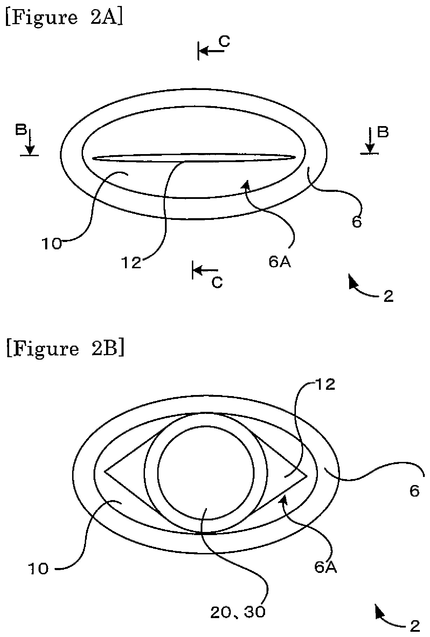

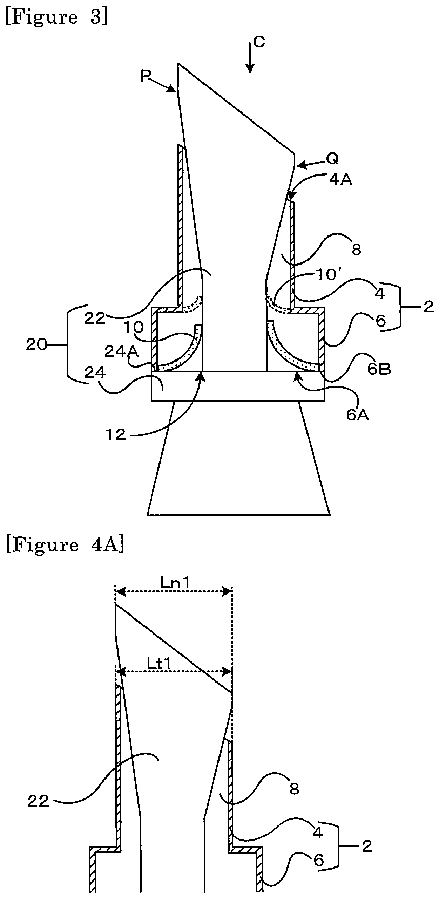

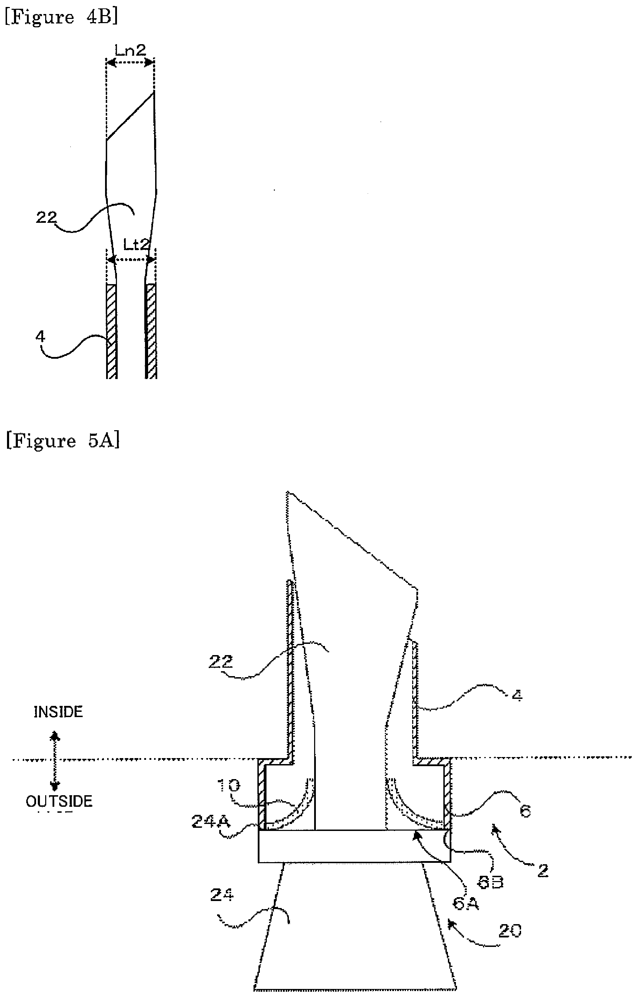

First, a trocar for ophthalmic surgery according to a first embodiment of the present invention will be described with reference to Figures 1 to 4B. Figure 1 is a perspective view showing an outer shape of a trocar for ophthalmic surgery according to a first embodiment of the present invention, where (a) is a view from the tip end and (b) is a view from the rear end. Figure 2A is a view taken along the arrows A-A in Figure 1(a), showing a side view of the trocar for ophthalmic surgery from the rear end. Figure 2B is a view showing a state that a knife or surgical instrument has been inserted into the trocar for ophthalmic surgery from the state shown in Figure 2A. Figure 3 is a cross-sectional view taken along the line B-B in Figure 2A, showing a side cross-section of the trocar for ophthalmic surgery and a state that a knife has been attached to the trocar for ophthalmic surgery. Figure 4A is a view showing the maximum longitudinal dimension Lt1 of the trocar for ophthalmic surgery and the maximum width dimension Ln1 of the attached knife. Figure 4B is a view showing the maximum lateral dimension Lt2 of the trocar for ophthalmic surgery and the maximum thickness dimension Ln2 of the attached knife.

A trocar for ophthalmic surgery 2 of this embodiment is constructed by integrally connecting a hollow insertion section 4 at a front side and a hollow main body section 6 at a rear side. An internal space 8 is formed from a front opening 4A of the insertion section 4 to a rear opening 6A of the main body section 6 (see Figure 3). In the trocar for ophthalmic surgery 2 of this embodiment, at least the insertion section 4 is elastic. The elastic material used to form the insertion section 4 can be any elastic rubber or resin material, including silicone.

The insertion section 4 and main body section 6 may have similar elasticity, or the main body section 6 may be formed from a harder material than the insertion section 4. It is also possible to vary the degree of elasticity in different regions of the insertion section 4, thereby allowing it to flexibly expand and contract while maintaining structural rigidity. The trocar for ophthalmic surgery 2 with different elasticity regions can be achieved, for example, by two-color molding.

The cross-sectional shape of the insertion section 4 and the front opening 4A are elongated and correspond to the cross-sectional shape of a cutting tip portion 22 of a knife 20 to be inserted. Meanwhile, the main body section 6 has an elliptical or circular cross-sectional shape with smaller differences in vertical and horizontal dimensions compared to the insertion section 4.

### <Dimensions of insertion section and cutting tip portion>

In this embodiment, the knife 20 is attached to the trocar for ophthalmic surgery 2 and used as an integrated set. As shown in Figure 4A, the maximum width dimension Ln1 of the cutting tip portion 22 of the knife 20 is equal to or greater than the maximum longitudinal dimension Lt1 of the insertion section 4 of the trocar for ophthalmic surgery 2. In other words, there is a relationship of Ln1 ≧ Lt. Similarly, as shown in Figure 4B, the maximum width dimension Ln2 of the cutting tip portion 22 of the knife 20 is equal to or greater than the maximum lateral dimension Lt2 of the insertion section 4 of the trocar for ophthalmic surgery 2. In other words, there is a relationship of Ln2 ≧ Lt2.

Then, the maximum outer diameter of the cutting tip portion 22 of the knife 20 is larger than the maximum outer diameter of the insertion section 4 of the trocar for ophthalmic surgery 2 that covers the cutting tip portion 22. However, when the cutting tip portion 22 is inserted into the insertion section 4, the insertion section 4 can elastically expand. Therefore, the cutting tip portion 22 of the knife 20 passes through the insertion section 4, thereby allowing the cutting tip portion 22 to protrude from the front opening 4A of the insertion section 4. The internal dimensions of the main body section 6 are larger than the external dimensions of the cutting tip portion 22 of the knife 20, thereby preventing interference between them. Furthermore, the internal dimensions of the main body section 6 are larger than the external dimensions of a surgical instrument 30, as described below, thereby preventing interference between them.

When removing the knife 20 from the trocar for ophthalmic surgery 2, just as when inserting the knife 20 into the trocar for ophthalmic surgery 2, the insertion section 4 elastically expands, causing the cutting tip portion 22 of the knife 20 to move rearward within the insertion section 4, thereby allowing only the knife 20 to be removed from the rear opening 6A of the main body section 6.

In this embodiment, the cutting tip portion 22 of the knife 20 does not have an edge formed in the area that comes into contact with an inner surface of the insertion section 4 when the knife 20 passes through the insertion section 4 (see arrows P and Q in Figure 3). The area that comes into contact with the inner surface of the insertion section 4, indicated by arrows P and Q in Figure 3, preferably has a smoothly curved shape.

In this embodiment, since the knife 20 has no edge in the area that comes into contact with the inner surface of the insertion section 4, the knife 20 can be inserted and removed smoothly without damaging the inner surface of the insertion section 4.

As described above, the maximum width dimension Ln1 of the cutting tip portion 22 is equal to or greater than the maximum longitudinal dimension Lt1 of an outer shape of the insertion section 4, and the maximum thickness dimension Ln2 of the cutting tip portion 22 is equal to or greater than the maximum lateral dimension Lt2 of an outer shape of the insertion section 4. This ensures the following:
when the knife 20 is placed in the internal space 8 so that the cutting tip portion 22 protrudes from the front opening 4A, the outline of the insertion section 4 is hidden by an outline of the cutting tip portion 22 when viewed from the distal end of the cutting tip portion 22 (see arrow C in Figure 3).

### <Valve formed of elastic thin film>

As shown in Figures 2A and 3, a valve 12 formed of an elastic thin film 10 is provided near the rear opening 6A in the main body section 6. The elastic thin film 10 can be formed from any elastic rubber or resin material, including silicone. In the illustrated example, a linear slit in the elastic thin film 10 forms an opening, which functions as the valve 12. However, the elastic thin film 10 is not limited to being formed near the rear opening 6A of the main body section 6. For example, as shown by the dotted line in Figure 3, the elastic thin film 10' can also be formed in the boundary region between the insertion section 4 and the main body section 6. Furthermore, the elastic thin films 10, 10' can also be formed both near the rear opening 6A of the main body section 6 and in the boundary region between the insertion section 4 and the main body section 6.

When the knife 20 or the surgical instrument 30 is not inserted into the trocar for ophthalmic surgery 2, the opening of the valve 12 remains generally closed, thereby preventing aqueous humor from leaking from the eyeball. On the other hand, even when the knife 20 or the surgical instrument 30 is inserted into the trocar for ophthalmic surgery 2, the opening of the valve 12 deforms to conform to the outer shape of the inserted knife 20 or the surgical instrument 30, thereby maintaining a tight seal as much as possible, as shown in Figure 2B. This allows for appropriate prevention of aqueous humor from leaking from within the eyeball, even when the knife 20 or the surgical instrument 30 is inserted into the trocar for ophthalmic surgery 2. Considering the strength when the knife 20 or the surgical instrument 30 is inserted, the elastic thin film 10 can be approximately 0.3 to 1.5 mm thick, for example.

As described above, in this embodiment, the valve 12 formed of the elastic thin film 10 is provided within the main body section 6. When the knife 20 or the surgical instrument 30 is inserted into the main body section 6 through the rear opening 6A, the opening of the valve 12 expands according to the outer shape of the knife 20 or the surgical instrument 30. When the knife 20 or the surgical instrument 30 is removed from the rear opening 6A, the opening of the valve 12 closes.

In this way, the elastic thin film 10 maintains a sealed state both when the knife 20 or the surgical instrument 30 is inserted and after it is removed, thereby reliably preventing aqueous humor from leaking from the interior of the eyeball.

In the illustrated example, the opening is formed of a linear slit in the elastic thin film 10, but this is not limited thereto. For example, the slit may meander or zigzag relative to the extension direction. The angle of the slit relative to the extension direction can also be any angle, up to a maximum of perpendicular. The slit shape may be a series of connected straight lines, a series of connected curves, or a combination of straight and curved lines. In either case, it is preferable to adopt a shape that provides high sealing and tracking capabilities depending on the outer shape of the inserted member.

### <Method of using the trocar for ophthalmic surgery>

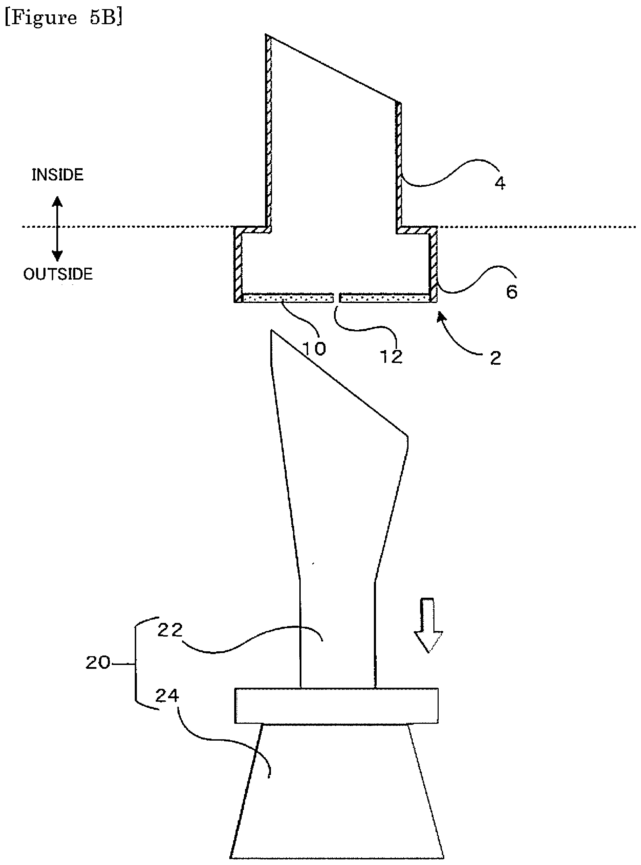

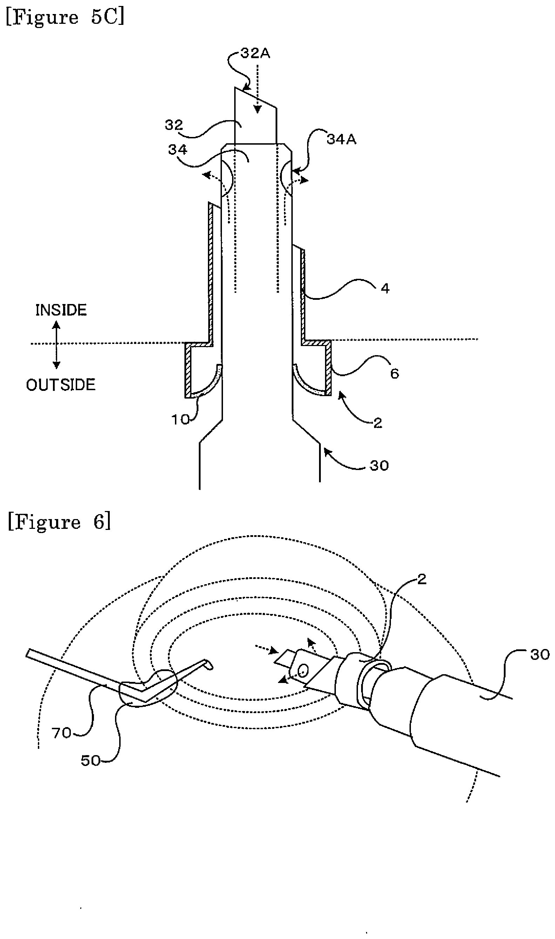

Next, with reference to Figures 5A to 5C, a method of using the trocar for ophthalmic surgery 2 according to this embodiment will be described. Figure 5A is a schematic view showing a state that the insertion section of the trocar for ophthalmic surgery has been inserted into the interior of the eyeball by inserting the cutting tip portion of the attached knife into the interior of the eyeball. Figure 5B is a schematic view showing a state that only the knife has been removed from the state shown in Figure 5A. Figure 5C is a schematic view showing insertion of a surgical instrument into the trocar for ophthalmic surgery with the insertion section inserted inside the eyeball to perform surgery.

As described above, when the knife 20 is placed in the internal space 8 of the trocar for ophthalmic surgery 2 so that the cutting tip portion 22 protrudes from the front opening 4A of the insertion section 4, the outline of the cutting tip portion 22 hides the outline of the insertion section 4 when viewed from the distal end of the cutting tip portion 22. Therefore, when the cutting tip portion 22 is inserted into the eyeball with the trocar for ophthalmic surgery 2 integrated with the knife 20, the insertion section 4 is also inserted into the eyeball together with the cutting tip portion 22 (see Figure 5A).

As described above, the interior of the main body section 6 of the trocar for ophthalmic surgery 2 is larger than the outer shape of the cutting tip portion 22 of the knife 20. At least a portion of the outer shape of the main body 24 of the knife 20 is larger than the outer shape of the main body section 6. As a result, an abutment surface 6B that abuts against a front surface 24A of the main body 24 of the knife 20 is formed at a rear side of the main body section 6 of the trocar for ophthalmic surgery 2. As a result, when the cutting tip portion 22 is inserted into the interior of the eyeball, the insertion section 4 is pushed by the main body 24 of the knife 20 via the abutment surface 6B, and the insertion section 4 is inserted into the interior of the eyeball together with the cutting tip portion 22.

In this way, when the cutting tip portion 22 is inserted into the interior of the eyeball, the insertion section 4 is also pushed toward the insertion side via the abutment surface 6B, thereby ensuring that the insertion section 4 is inserted into the interior of the eyeball together with the cutting tip portion 22.

As described above, when viewed from the distal end of the cutting tip portion 22, the outline of the insertion section 4 is hidden by the outline of the cutting tip portion 22. However, even if the outer shape of the insertion section 4 were to extend slightly outward from the outer shape of the cutting tip portion 22, interference with the eyeball would be extremely limited, and the insertion section 4 of the trocar for ophthalmic surgery 2 would be inserted relatively smoothly into the eyeball together with the cutting tip portion 22 of the knife 20. In particular, with regard to the shorter side of the insertion section 4, even if the outer shape of the insertion section 4 extends slightly outward from the outer shape of the cutting tip portion 22, the degree of contact between the cutting tip portion 22 and the insertion section 4 is high, and the insertion section 4 would also be inserted relatively smoothly into the eyeball.

Furthermore, since the insertion section 4 is also pushed from behind via the abutment surface 6B, more reliably inserting the insertion section 4 into the eyeball. Considering these factors, it is said that if the outline of the insertion section 4 is generally hidden by the outline of the cutting tip portion 22 when viewed from the distal end of the cutting tip portion 22, the insertion section 4 can be inserted inside the eyeball.

Next, by removing only the knife 20 from the rear opening 6A of the trocar for ophthalmic surgery 2, an installation state is achieved in which the insertion section 4 is located inside the eyeball and the main body section 6 is located outside the eyeball, as shown in Figure 5B. At this time, the insertion section 4 of the trocar for ophthalmic surgery 2 elastically expands, and there is no edge in the area of the knife 20 that contacts the inner surface of the insertion section 4, thereby allowing only the knife 20 to be smoothly removed. This results in an installation state that the insertion section 4 of the trocar for ophthalmic surgery 2 is located inside the eyeball and the main body section 6 is located outside the eyeball, as shown in Figure 5B.

Basically, the surgeon simply inserts the cutting tip portion 22 of the knife 20, which is integrated with the insertion section 4, into the eyeball. This allows the insertion section 4 to be reliably inserted into the eyeball without relying on the surgeon's skill, thereby shortening the time required for installation and improving work efficiency. In the installed state after the knife 20 is removed, the valve 12, which closes the opening of the elastic thin film 10 located near the rear opening 6A, is in a closed state, thereby preventing aqueous humor from leaking from the eyeball.

In this installed state, the surgical instrument 30 is inserted through the rear opening 6A of the trocar for ophthalmic surgery 2. When the surgical instrument 30 is inserted, the opening of the elastic thin film 10 located near the rear opening 6A deforms according to the outer shape of the surgical instrument 30, thereby allowing the surgical instrument 30 to pass through. At this time, the deformation maintains a seal as much as possible, thereby preventing aqueous humor from leaking from the eyeball.

In the installed state, when the surgical instrument 30 is not present, the insertion section 4 of the trocar for ophthalmic surgery 2 is pushed from the outside by the pressure of aqueous humor and ocular tissue, which results in a nearly collapsed internal space. When the surgical instrument 30 is inserted into the insertion section 4, the insertion section 4 elastically expands to conform to the outer shape of the surgical instrument 30. At this time, the portion of the eyeball covering the insertion section 4 also expands in response to the expansion of the insertion section 4. Since the insertion section 4 does not move in the insertion direction, the eyeball is not damaged. The eyeball can expand in response to the expansion of the insertion section 4, thereby preventing the outflow of aqueous humor from the interior of the eyeball. This allows the distal end of the surgical instrument 30 to be positioned within the eyeball, thereby allowing surgery to be performed.

The trocar for ophthalmic surgery 2 of the first embodiment is designed to allow the insertion of a surgical instrument 30, which is an ultrasonic handpiece with an irrigation sleeve. As shown in Figure 5C, the surgical instrument 30 is primarily composed of an ultrasonic transducer 32 and an irrigation sleeve 34 covering the outer surface of the ultrasonic transducer 32. The ultrasonic transducer 32, for example, irradiates the crystalline lens with ultrasound to create a fragmented area. Irrigation fluid flows between the inner surface of the irrigation sleeve 34 and the outer surface of the ultrasonic transducer 32, and flows into the eyeball through an opening 34A at the tip. The fragmented area is aspirated from an opening 32A at the tip of the ultrasonic transducer 32 together with aqueous humor and irrigation fluid.

The ultrasonic transducer 32 becomes hot during operation, however since irrigation fluid flows along its exterior, the temperature of the outer surface of the irrigation sleeve 34 covering it does not increase. Therefore, the trocar for ophthalmic surgery 2 according to the first embodiment can be formed from a material with or without heat resistance.

An outer diameter of the surgical instrument 30 that passes through this insertion section 4 can be, for example, in the range of 1.5 mm to 4 mm. Even if a metal trocar that could pass a surgical instrument 30 of this size is created, it would not be possible to insert the insertion section into the eyeball without damaging the eyeball. As described above, by using a set consisting of the trocar for ophthalmic surgery 2 with the elastic insertion section 4 and the knife 20 integrated therewith, it is possible for the insertion section 4 that is large enough to pass the surgical instrument 30 with an outer diameter of 1.5 mm to 4 mm to be placed inside the eyeball.

After the surgical procedure using the surgical instrument 30 is completed, when the surgical instrument 30 is removed, the elastically deformed insertion section 4 is pushed from the outside by the pressure of the aqueous humor and ocular tissue, returning to its original shape. Since the insertion section 4 does not move even when the surgical instrument 30 is removed, the problem of aqueous humor leaking out of the eye, which was a conventional problem, is avoided.

As described above, the trocar for ophthalmic surgery 2 according to the first embodiment of the present invention is a trocar for ophthalmic surgery 2 having a hollow 4 insertion section at a front side connected to a hollow main body section 6 at a rear side, forming an internal space 8 from a front opening 4A of the insertion section 4 to a rear opening 6A of the main body section 6, wherein at least the insertion section4 is elastic, wherein when a knife 20 is placed in the internal space 8 so that a cutting tip portion 22 of the knife 20 protrudes from the front opening 4A, and the knife 20 is viewed from the distal end of the cutting tip portion 22, an outline of the insertion section 4 is generally hidden by an outline of the cutting tip portion 22, when the cutting tip portion 22 is inserted into an interior of the eyeball, the insertion section 4 is also inserted into the interior of the eyeball together with the cutting tip portion 22, and by removing only the knife 20 from the rear opening 6A, an installed state is established in which the insertion section 4 is located inside the eyeball and the main body section 6 is located outside the eyeball, wherein the insertion section 4 expands, thereby allowing the cutting tip portion 22 to pass through the insertion section 4, wherein in the installed state, when a surgical instrument 30 is inserted into the insertion section 4 through the rear opening 4A, the insertion section 4 expands and a tip of the surgical instrument 30 is inserted into the eyeball; and when the surgical instrument 30 is removed from the rear opening 6A, the expanded insertion section 4 returns to its original shape.

The outer shape of the cutting tip portion 22 of the knife 20 is larger than the outer shape of the insertion section 4 of the trocar for ophthalmic surgery 2, however the insertion section 4 elastically expands, thereby allowing the cutting tip portion 22 to pass through the insertion section 4 and protrude from the front opening 4A of the insertion section 4. When viewed from the distal end of the cutting tip portion 22, the outline of the insertion section 4 is generally hidden by the outline of the cutting tip portion 22. Therefore, when the cutting tip portion 22 is inserted into the eyeball, the insertion section 4 can be inserted into the eyeball together with the cutting tip portion 22. Then, by removing only the knife 20, an installation state can be easily achieved in which the insertion section 4 is located inside the eyeball and the main body section 6 is located outside the eyeball. Furthermore, when removing the knife 20, the insertion section 4 elastically deforms, so the knife can be removed while the insertion section 4 remains inside the eyeball.

Basically, the knife 20, which is integrated with the insertion section 4, is simply inserted into the eyeball, so the operation does not depend on the surgeon's skill. This allows the insertion section to be reliably inserted into the eyeball, shortens the installation time, and improves work efficiency.

When a relatively large surgical instrument 30 is inserted or removed in the installed state, the insertion section 4 elastically deforms but does not move in the insertion direction, allowing the eyeball to follow, preventing aqueous humor from leaking from the interior of the eyeball. In this way, surgical instruments 30, such as surgical handpieces, can be safely and reliably inserted and removed, preventing injuries such as crushing the wound or the burns.

This provides a trocar for ophthalmic surgery that allows a surgical instrument with larger outer dimensions than forceps to be reliably inserted into the eyeball, regardless of the surgeon's skill.

It is noted that in this embodiment, the insertion section 4 of the trocar for ophthalmic surgery 2 can be maintained in the installed state within the eyeball, and other surgical instruments can be inserted in addition to the surgical instrument 30 that is an ultrasonic handpiece with an irrigation sleeve for cataract surgery, to continue performing vitreous surgery etc. Furthermore, the trocar for ophthalmic surgery 2 according to this embodiment can be applied not only to cataract surgery, but also to glaucoma surgery, corneal transplants, and various other eye surgeries.

### (Trocar system including trocar for ophthalmic surgery according to first embodiment of the present invention)

Next, with reference to Figure 6, a trocar system including trocar for ophthalmic surgery including the trocar for ophthalmic surgery 2 according to the first embodiment will be briefly described. Figure 6 is a schematic view showing performance of eye surgery using a trocar system including trocar for ophthalmic surgery including the trocar for ophthalmic surgery according to the first embodiment of the present invention.

This trocar system including trocar for ophthalmic surgery comprises at least the trocar for ophthalmic surgery 2 according to the first embodiment, an ultrasonic handpiece surgical instrument 30, and an ultrasonic transducer 32 having an opening 32A for aspirating irrigation fluid and the fragmented portion, and an irrigation sleeve 34 arranged to cover the outer surface of the ultrasonic transducer 32 for supplying irrigation fluid.

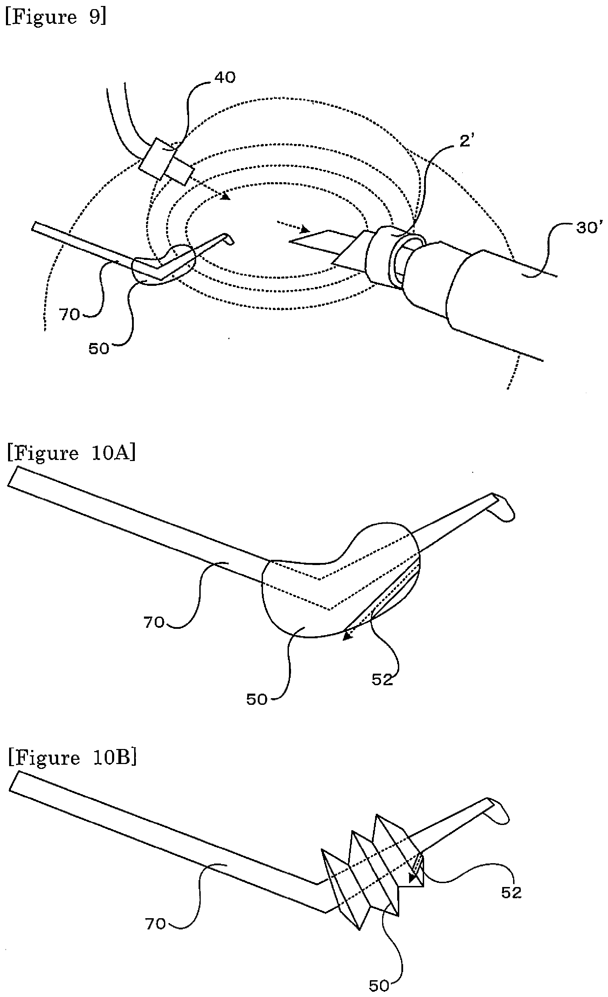

Figure 6 shows a surgical procedure using a hook-shaped surgical auxiliary instrument 70. The surgical auxiliary instrument 70 is equipped with a side port seal 50 to prevent aqueous humor from leaking from the eyeball. The side port seal 50 will be described in detail later with reference to Figures 10A and 10B.

The trocar system including trocar for ophthalmic surgery comprising at least the trocar for ophthalmic surgery 2 of the first embodiment and the surgical instrument 30, and the ultrasonic handpiece capable of supplying irrigation fluid, allows safe and reliable insertion and removal of surgical instruments regardless of the surgeon's skill, thereby preventing injuries such as the crushing and the burns to the wound and enabling reliable cataract surgery.

### (Trocar for Ophthalmic Surgery according to second embodiment of the present invention)

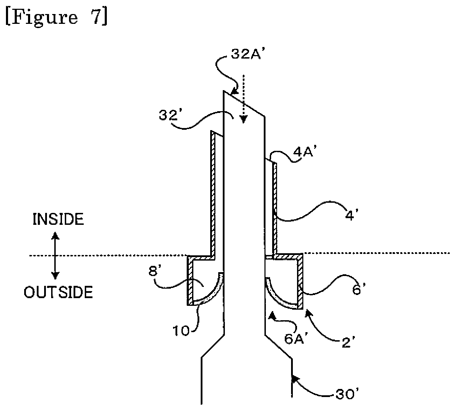

Next, a trocar for ophthalmic surgery according to a second embodiment of the present invention will be described with reference to Figures 7 to 8B.

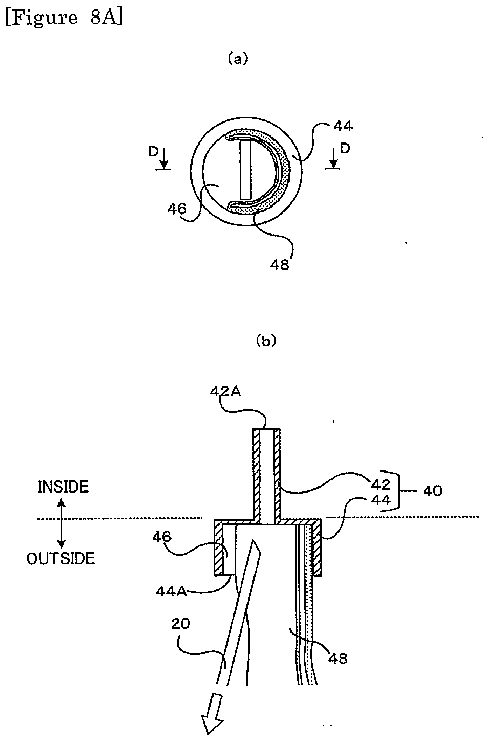

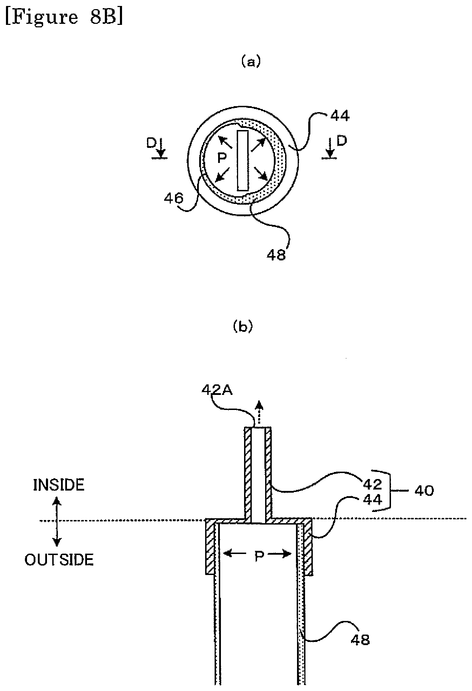

Figure 7 is a side cross-sectional view of a trocar for ophthalmic surgery according to a second embodiment of the present invention, particularly showing an insertion section inserted inside the eyeball and insertion of surgical instruments to perform surgery. Figures 8A and 8B are views showing an example of an infusion trocar to be used with the trocar for ophthalmic surgery according to the second embodiment of the present invention, Figures 8A shows the irrigation fluid tube attached to the internal space of the main body in a collapsed state, and Figure 8B shows supply of irrigation fluid into a folded irrigation fluid tube to supply fluid into the eyeball. In Figures. 8A and 8B, (a) is a view of an infusion trocar 40 from a rear opening 44A of a main body section 44, and (b) is a side cross-sectional view showing section D-D of (a).

A trocar for ophthalmic surgery 2' according to a second embodiment of the present invention has a structure similar to that of the first embodiment. However, the trocar for ophthalmic surgery 2' according to this embodiment is designed to accommodate the insertion of a surgical instrument 30', which is an ultrasonic handpiece without an irrigation sleeve. The absence of the irrigation sleeve reduces the overall size of the surgical instrument 30'. Therefore, the overall size of the trocar for ophthalmic surgery 2' according to the second embodiment can be made smaller than that of the first embodiment. This allows for easier insertion of the insertion section 4 into the eyeball.

However, although the ultrasonic transducer 32 becomes hot during operation, since there is no irrigation sleeve or other covering, the trocar for ophthalmic surgery 2' according to the second embodiment is preferably made of a heat resistant material.

In this embodiment, an infusion trocar 40 is used to supply irrigation fluid into the eye during eye surgery. The infusion trocar 40 has a structure similar to that of the trocar for ophthalmic surgery 2 described above. In other words, a hollow insertion section 42 at a front side and a hollow main body section 44 at a rear side are connected, forming an internal space 46 from a front opening 42A of the insertion section 42 to a rear opening 44A of the main body section 44, with at least the insertion section 42 being elastic. In a manner similar to the trocar for ophthalmic surgery 2 described above, the infusion trocar 40 can be placed in an installation state that the insertion section 42 is placed inside the eyeball and the main body section 44 is placed outside the eyeball.

The infusion trocar 40 also has an irrigation fluid tube 48 attached to an inner space of the internal space 46 of the main body section 44. As shown in Figure 8A, when the irrigation fluid tube 48 is folded, there is a space within the main body section 44 for placing a knife 20. Figure 8A shows that the knife 20 used to insert the insertion section 42 into the eyeball is removed from the infusion trocar 40.

The irrigation fluid tube 48 is made of a highly waterproof, elastic resin or rubber material. When irrigation fluid is supplied from the irrigation fluid supply source after the knife 20 is withdrawn from the rear end opening 44A, the irrigation fluid tube 48, which was folded due to the fluid pressure of the irrigation fluid, expands and comes into contact with the entire inner surface of the main body section 44, thereby forming a sealed state, as shown in Figure 8B. This allows irrigation fluid to be reliably supplied into the eyeball while suppressing leakage. The irrigation fluid tube 48, except for the area where the knife 20 is inserted, preferably has a thickness that provides sufficient rigidity to maintain a seal. The portion of the irrigation fluid tube 48 located in the area where the knife 20 is inserted is preferably thinner than other areas to ensure sufficient flexibility.

In this way, the infusion trocar 40 allows the insertion section 42 to be reliably inserted into the eyeball regardless of the surgeon's skill level. Furthermore, the pre-attached irrigation fluid tube 48 allows for the supply of irrigation fluid with minimal effort. The folded, elastic irrigation fluid tube 48 allows the knife 20 to be inserted and removed without interference when the trocar is attached. Simply supplying irrigation fluid creates a seal with hydraulic pressure, thereby ensuring that the irrigation fluid is reliably supplied into the eyeball.

### (Trocar system including trocar for ophthalmic surgery according to second embodiment of the present invention)

Next, with reference to Figure 9, a brief description will be given of a trocar system including trocar for ophthalmic surgery including the trocar for ophthalmic surgery 2 according to the second embodiment of the present invention. Figure 9 is a view schematically showing performance of eye surgery using a trocar system including trocar for ophthalmic surgery including a trocar for ophthalmic surgery and the infusion trocar according to the second embodiment of the present invention.

This trocar system including trocar for ophthalmic surgery comprises at least the trocar for ophthalmic surgery 2 according to the second embodiment described above, a surgical instrument 30' which is an ultrasonic handpiece including an ultrasonic transducer 32' having an opening 32A' for aspirating irrigation fluid and a fragmented portion, and the infusion trocar 40 for providing irrigation fluid, and the trocar for ophthalmic surgery 2' is heat resistant.

Figure 9 also shows that surgery is performed using a hook-shaped surgical auxiliary instrument 70. The surgical auxiliary instrument 70 is equipped with a side port seal 50 for preventing aqueous humor from leaking from the eyeball. The side port seal 50 will be described in detail later with reference to Figures 10A and 10B.

In this trocar system, the surgical instrument 30' which is an ultrasonic handpiece does not have an irrigation sleeve arranged to cover the outer surface of the ultrasonic transducer 32', thereby allowing the overall size of the surgical instrument 30' to be reduced. This allows for a smaller external size for the trocar for ophthalmic surgery 2', making it easier to insert the trocar for ophthalmic surgery 2' into the eyeball. Furthermore, the separate infusion trocar 40 allows for more flexible adjustment of the flow of irrigation fluid within the eyeball.

### (Side Port Seal)

Next, with reference to Figures 10A and 10B, a side port seal attached to a surgical auxiliary instrument will be explained. Figure 10A is a perspective view schematically showing an example of a side port seal attached to a surgical auxiliary instrument. Figure 10B is a perspective view schematically showing another example of a side port seal attached to a surgical auxiliary instrument.

A side port seal 50 attached to a hook-shaped surgical auxiliary instrument 70 is elastic and stretchable. Therefore, it is preferable to form the side port seal 50 from any elastic rubber or resin material, such as silicone. Since the surgical auxiliary instrument 70 has a small external size, a side port can be created by conventional surgeon procedures and the tip of the surgical auxiliary instrument 70 can be inserted into the interior of the eyeball without using a trocar as described above. At this time, the side port seal 50 attached to the surgical auxiliary instrument 70 is positioned at the boundary between the interior and exterior of the eyeball.

In the example shown in Figure 10A, the side port seal 50 has a smoothly curved shape, while in the example shown in Figure 10B, the side port seal 50 has a stretchable bellows shape. It is preferable that the side port seal 50 have an optimal shape depending on the shape of the surgical auxiliary instrument 70. In either case, the elastic side port seal 50 can prevent aqueous humor and other fluids from leaking from the eyeball.

A trocar system including trocar for ophthalmic surgery including the trocar for ophthalmic surgery 2, 2' according to the first or second embodiment further includes the elastic, stretchable side port seal 50 that covers the exterior of the surgical auxiliary instrument 70. When the tip of the surgical auxiliary instrument 70 is inserted into the eyeball through the side port provided in the eyeball, the side port seal 50 can prevent aqueous humor and other fluids from leaking from the side port.

Accordingly, the small external shape of the surgical auxiliary instrument 70 makes it possible to provide a side port in the eyeball without using a trocar with a knife. When the tip of the surgical auxiliary instrument 70 is inserted into the eyeball, the side port seal 50 can reliably prevent aqueous humor and other fluids from leaking from the side port.

Regardless of the sealing effect of the side port seal 50, even if a small amount of aqueous humor or other fluid leaks from the side port, a guide groove 52 is formed to drain the effluent to an area that is less likely to affect the surgery. The side port seal 50 is formed with the guide groove 52 that guides the flow of aqueous humor and other substances flowing out of the side port, thereby allowing the outflowing aqueous humor to be disposed without interfering with surgery.

### (Contact Lens)

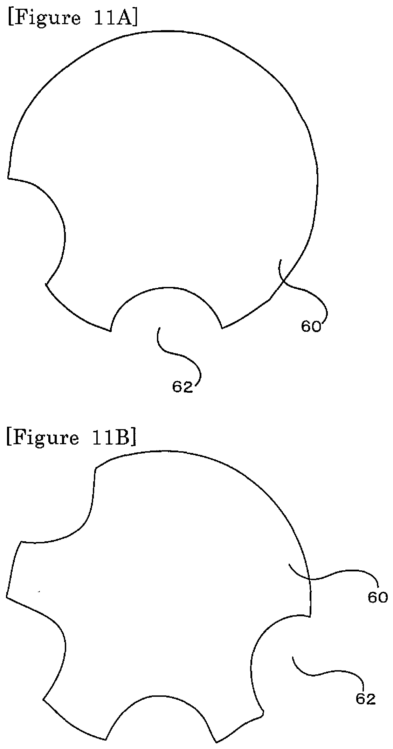

Next, with reference to Figures 11A and 11B, we will explain contact lenses that cover the eyeball during surgery. Figure 11A is a perspective view schematically showing an example of a contact lens that covers the eyeball during surgery, used in conjunction with the trocar for ophthalmic surgery according to the first embodiment of the present invention. Figure 11B is a perspective view schematically showing an example of a contact lens that covers the eyeball during surgery and is used together with the trocar for ophthalmic surgery according to the second embodiment of the present invention.

During surgery, if the surface of the eyeball dries out, it becomes cloudy, thereby making it difficult for the surgeon to see inside the eyeball. To address this issue, a practical method involves covering the eyeball with a hydrophobic or water-repellent contact lens; however, this cannot be used because it interferes with the above-described Trocars for Ophthalmic Surgery 2, 2', infusion trocar 40, surgical auxiliary instrument 70 or the like.

For this reason, the trocar system including trocar for ophthalmic surgery including the trocars for ophthalmic surgery 2, 2' according to the first and second embodiments is equipped with a contact lens 60 as shown in Figures 11A and 11B. More specifically, the contact lens 60 used with the trocars for ophthalmic surgery 2, 2' according to the first and second embodiments covers the eyeball during surgery, is hydrophobic or water-repellent, and has a cutout portion 62 to avoid interference with instruments inserted into the eyeball.

The contact lens 60 shown in Figure 11A is a contact lens provided in a trocar system including trocar for ophthalmic surgery including the trocar for ophthalmic surgery 2 according to the first embodiment, and has two cutouts 62 corresponding to the trocar for ophthalmic surgery 2 and surgical auxiliary instrument 70. The contact lens 60 shown in Figure 11B is a contact lens provided in a trocar system including trocar for ophthalmic surgery including the trocar for ophthalmic surgery 2 according to the second embodiment, and has four cutouts 62 corresponding to the trocar for ophthalmic surgery 2', infusion trocar 40, surgical auxiliary instrument 70, and other components.

As described above, by covering the eyeball with the hydrophobic or water-repellent contact lens 60 having cutouts 62, visibility of the inside of the eyeball can be ensured during surgery without interfering with surgical components. It is noted that the above embodiment is merely an example up to the point of opening. Any number of cutouts can be provided and the lens can be made of any material as long as they do not interfere with the trocar.

### (General Description)

(1) The 1st aspect of the present invention is a trocar for ophthalmic surgery having a hollow insertion section at a front side connected to a hollow main body section at a rear side, forming an internal space from a front opening of the insertion section to a rear opening of the main body section,
   wherein at least the insertion section is elastic,
   wherein when a knife is placed in the internal space so that a cutting tip portion of the knife protrudes from the front opening, and the knife is viewed from the distal end of the cutting tip portion, an outline of the insertion section is generally hidden by an outline of the cutting tip portion, when the cutting tip portion is inserted into an interior of the eyeball, the insertion section is also inserted into the interior of the eyeball together with the cutting tip portion, and by removing only the knife from the rear opening, an installed state is established in which the insertion section is located inside the eyeball and the main body section is located outside the eyeball,
   wherein the insertion section expands, thereby allowing the cutting tip portion to pass through the insertion section,
   wherein in the installed state, when a surgical instrument is inserted into the insertion section through the rear opening, the insertion section expands and a tip of the surgical instrument is inserted into the eyeball; and when the surgical instrument is removed from the rear opening, the expanded insertion section returns to its original shape.
(2) The 2nd aspect of the present invention is the trocar for ophthalmic surgery according to 1st aspect,
   wherein an outer diameter of the surgical instrument passing through the insertion section is in the range of 1.5 mm to 4 mm.
(3) The 3rd aspect of the present invention is the trocar for ophthalmic surgery according to 1st aspect,
   wherein a maximum width dimension of the cutting tip portion is equal to or greater than a maximum longitudinal dimension of an outer shape of the insertion section, and a maximum thickness dimension of the cutting tip portion is equal to or greater than a maximum lateral dimension of an outer shape of the insertion section.
(4) The 4th aspect of the present invention is the trocar for ophthalmic surgery according to 1st aspect,
   wherein a rear end of the main body has an abutment surface that abuts against a front surface of a main body of the knife, and when the cutting tip portion is inserted into the interior of the eyeball, the main body of the knife presses against the abutment surface, causing the insertion section to be inserted into the interior of the eyeball together with the cutting tip portion.
(5) The 5th aspect of the present invention is the trocar for ophthalmic surgery according to 1st aspect,
   wherein the cutting tip portion does not have an edge formed in the area that contacts an inner surface of the insertion section when the knife passes through the insertion section.
(6) The 6th aspect of the present invention is the trocar for ophthalmic surgery according to 1st aspect,
   wherein the main body section is provided with a valve formed of an elastic thin film,
   when the knife or the surgical instrument is inserted into the main body section from the rear opening, an opening of the valve expands according to an outer shape of the knife or the surgical instrument, and when the knife or the surgical instrument is removed from the rear opening, the opening of the valve closes.
(7) The 7th aspect of the present invention is a trocar system including trocar for ophthalmic surgery comprising:
   the trocar for ophthalmic surgery according to any one of Claims 1 to 61
   the surgical instrument being an ultrasonic handpiece including an ultrasonic transducer having a suction port for aspirating irrigation fluid and fragmentation sections, and an irrigation sleeve arranged to cover the outer surface of the ultrasonic transducer for supplying irrigation fluid.
(8) The 8th aspect of the present invention is a trocar system including trocar for ophthalmic surgery comprising:
   the trocar for ophthalmic surgery according to any one of 1st to 6th aspects,
   the surgical instrument being an ultrasonic handpiece including an ultrasonic transducer having a suction port for aspirating irrigation fluid and fragmentation sections, and
   an infusion trocar for supplying irrigation fluid,
   wherein the trocar for ophthalmic surgery is heat resistant.
(9) The 9th aspect of the present invention is the trocar system including trocar for ophthalmic surgery according to 8th aspect,
   wherein the infusion trocar has a structure similar to that of the trocar for ophthalmic surgery according to Claim 1, further comprising an irrigation fluid tube attached to an internal space of the main body section in the internal space,
   wherein when the irrigation fluid tube is folded, there is a space within the main body section for placing the knife,
   wherein when irrigation fluid is supplied after the knife is removed from the rear opening the irrigation fluid tube expands due to the hydraulic pressure of the irrigation fluid, contacting the entire inner surface of the main body section, thereby forming a sealing state.
(10) The 10th aspect of the present invention is the trocar system including trocar for ophthalmic surgery according to 7th aspect,
   further comprising an elastic, stretchable side port seal for covering the exterior of a surgical auxiliary instrument,
   wherein when the tip of the surgical auxiliary instrument is inserted into the eyeball through a side port provided in the eyeball, the side port seal prevents aqueous humor from leaking out of the side port.
(11) The 11th aspect of the present invention is the trocar system including trocar for ophthalmic surgery according to 10th aspect,
   wherein a groove for guiding the flow of aqueous humor flowing out from the side port is formed in the side port seal.
(12) The 12th aspect of the present invention is the trocar system including trocar for ophthalmic surgery according to 7th aspect,
   further comprising a hydrophobic or water-repellent contact lens having a cutout for covering the eyeball during surgery and avoiding interference with instruments inserted into the eyeball.
(13) The 13th aspect of the present invention is the trocar system including trocar for ophthalmic surgery according to 8th aspect,
   further comprising an elastic, stretchable side port seal for covering the exterior of a surgical auxiliary instrument,
   wherein the side port seal prevents the outflow of aqueous humor from the side port when the surgical auxiliary instrument is inserted into the eyeball through the side port provided in the eyeball.
(14) The 14th aspect of the present invention is the trocar system including trocar for ophthalmic surgery according to 13th aspect,
   wherein 1 a guide groove for guiding the flow of aqueous humor flowing out from the side port is formed in the side port seal.
(15) The 15th aspect of the present invention is the trocar system including trocar for ophthalmic surgery according to 8th aspect,
   further comprising a hydrophobic or water-repellent contact lens having a cutout for covering the eyeball during surgery and avoiding interference with instruments inserted into the eyeball.

Although embodiments of the present invention have been described, the disclosed contents may be varied in the details of the configuration, and changes in the combination and order of elements in the embodiments may be realized without departing from the scope and spirit of the claimed invention.

### [Explanation of Symbols]

2, 2' Trocar for Ophthalmic Surgery
4, 4' Insertion Section
4A, 4A' Front Opening
6, 6' Main Body Section
6A, 6A' Rear Opening
8, 8' Internal Space
10, 10' Elastic Thin Film
12 Valve
20 Knife
22 Cutting Tip Portion
24 Main Body
30, 30' Surgical Instrument
32 Ultrasonic Transducer
32A Opening
34 Irrigation Sleeve
34A Opening
40 Infusion Trocar
42 Insertion Section
42A Front Opening
44 Main Body Section
44A Rear Opening
46 Internal Space
48 Irrigation Fluid Tube
50 Side Port Seal
52 Guide Groove
60 Contact Lens
70 Surgical Auxiliary Instrument

## Claims

1. A trocar for ophthalmic surgery having a hollow insertion section at a front side connected to a hollow main body section at a rear side, forming an internal space from a front opening of the insertion section to a rear opening of the main body section,
wherein at least the insertion section is elastic,
wherein when a knife is placed in the internal space so that a cutting tip portion of the knife protrudes from the front opening, and the knife is viewed from the distal end of the cutting tip portion, an outline of the insertion section is generally hidden by an outline of the cutting tip portion, when the cutting tip portion is inserted into an interior of the eyeball, the insertion section is also inserted into the interior of the eyeball together with the cutting tip portion, and by removing only the knife from the rear opening, an installed state is established in which the insertion section is located inside the eyeball and the main body section is located outside the eyeball,
wherein the insertion section expands, thereby allowing the cutting tip portion to pass through the insertion section,
wherein in the installed state, when a surgical instrument is inserted into the insertion section through the rear opening, the insertion section expands and a tip of the surgical instrument is inserted into the eyeball; and when the surgical instrument is removed from the rear opening, the expanded insertion section returns to its original shape.

2. The trocar for ophthalmic surgery according to Claim 1,
wherein an outer diameter of the surgical instrument passing through the insertion section is in the range of 1.5 mm to 4 mm.

3. The trocar for ophthalmic surgery according to Claim 1,
wherein a maximum width dimension of the cutting tip portion is equal to or greater than a maximum longitudinal dimension of an outer shape of the insertion section, and a maximum thickness dimension of the cutting tip portion is equal to or greater than a maximum lateral dimension of an outer shape of the insertion section.

4. The trocar for ophthalmic surgery according to Claim 1,
wherein a rear end of the main body has an abutment surface that abuts against a front surface of a main body of the knife, and when the cutting tip portion is inserted into the interior of the eyeball, the main body of the knife presses against the abutment surface, causing the insertion section to be inserted into the interior of the eyeball together with the cutting tip portion.

5. The trocar for ophthalmic surgery according to Claim 1,
wherein the cutting tip portion does not have an edge formed in the area that comes into contact with an inner surface of the insertion section when the knife passes through the insertion section.

6. The trocar for ophthalmic surgery according to Claim 1,
wherein the main body section is provided with a valve formed of an elastic thin film,
when the knife or the surgical instrument is inserted into the main body section from the rear opening, an opening of the valve expands according to an outer shape of the knife or the surgical instrument, and when the knife or the surgical instrument is removed from the rear opening, the opening of the valve closes.

7. A trocar system including trocar for ophthalmic surgery comprising:
the trocar for ophthalmic surgery according to any one of Claims 1 to 6l
the surgical instrument being an ultrasonic handpiece including an ultrasonic transducer having a suction port for aspirating irrigation fluid and fragmentation sections, and an irrigation sleeve arranged to cover the outer surface of the ultrasonic transducer for supplying irrigation fluid.

8. A trocar system including trocar for ophthalmic surgery comprising:
the trocar for ophthalmic surgery according to any one of Claims 1 to 6, the surgical instrument being an ultrasonic handpiece including an ultrasonic transducer having a suction port for aspirating irrigation fluid and fragmentation sections, and
an infusion trocar for supplying irrigation fluid,
wherein the trocar for ophthalmic surgery is heat resistant.

9. The trocar system including trocar for ophthalmic surgery according to Claim 8,
wherein the infusion trocar has a structure similar to that of the trocar for ophthalmic surgery according to Claim 1, further comprising an irrigation fluid tube attached to an internal space of the main body section in the internal space,
wherein when the irrigation fluid tube is folded, there is a space within the main body section for placing the knife,
wherein when irrigation fluid is supplied after the knife is removed from the rear opening the irrigation fluid tube expands due to the hydraulic pressure of the irrigation fluid, contacting the entire inner surface of the main body section, thereby forming a sealing state.

10. The trocar system including trocar for ophthalmic surgery according to Claim 7,
further comprising an elastic, stretchable side port seal for covering the exterior of a surgical auxiliary instrument,
wherein when the tip of the surgical auxiliary instrument is inserted into the eyeball through a side port provided in the eyeball, the side port seal prevents aqueous humor from leaking out of the side port.

11. The trocar system including trocar for ophthalmic surgery according to Claim 10,
wherein a groove for guiding the flow of aqueous humor flowing out from the side port is formed in the side port seal.

12. The trocar system including trocar for ophthalmic surgery according to Claim 7,
further comprising a hydrophobic or water-repellent contact lens having a cutout for covering the eyeball during surgery and avoiding interference with instruments inserted into the eyeball.

13. The trocar system including trocar for ophthalmic surgery according to Claim 8,
further comprising an elastic, stretchable side port seal for covering the exterior of a surgical auxiliary instrument,
wherein the side port seal prevents the outflow of aqueous humor from a side port when the surgical auxiliary instrument is inserted into the eyeball through the side port provided in the eyeball.

14. The trocar system including trocar for ophthalmic surgery according to Claim 13, wherein 1a guide groove for guiding the flow of aqueous humor flowing out from the side port is formed in the side port seal.

15. The trocar system including trocar for ophthalmic surgery according to Claim 8,
further comprising a hydrophobic or water-repellent contact lens having a cutout for covering the eyeball during surgery and avoiding interference with instruments inserted into the eyeball.
